# EUROPÄISCHE PATENTSCHRIFT

(11) **EP 0 918 793 B1**
(45) Veröffentlichungstag und Bekanntmachung des Hinweises auf die Patenterteilung: **17.04.2002**
(21) Anmeldenummer: 97936699.4
(22) Anmeldetag: 11.08.1997
(51) Int. Cl.: C07K 7/06

(54) **VERFAHREN ZUR INAKTIVIERUNG VON LIPIDUMHÜLLTEN VIREN MIT LIPOPEPTIDEN UND NEUE ANTIVIRALE LIPOPEPTIDE**
INACTIVATING PROCESS FOR LIPID ENVELOPPED VIRUS, AND NEW ANTIVIRAL LIPOPEPTIDES
PROCEDE D'INACTIVATION DE VIRUS A ENVELOPPE LIPIDIQUE ET NOUVEAUX LIPOPEPTIDES ANTIVIRALES

(30) Priorität: 12.08.1996 DE 19633684
(43) Veröffentlichungstag der Anmeldung: 02.06.1999
(73) Patentinhaber: Vollenbroich, Dirk, 12049 Berlin (DE); Vater, Joachim, 14109 Berlin (DE); Pauli, Georg, 10785 Berlin (DE); Kamp, Roza Maria, 14167 Berlin (DE)
(72) Erfinder: Vollenbroich, Dirk, 12049 Berlin (DE); Vater, Joachim, 14109 Berlin (DE); Pauli, Georg, 10785 Berlin (DE); Kamp, Roza Maria, 14167 Berlin (DE)
(74) Vertreter: Ziebig, Marlene, Dr. Dipl.-Chem.
(86) Internationale Anmeldenummer: EP9704353
(87) Internationale Veröffentlichungsnummer: WO9806744

(56) Entgegenhaltungen:
- WO-A-95/32990
- DE-A- 19 521 938
- US-A- 4 841 023
- N. NARUSE ET AL.: "Pumilacidin, a Complex of new Antiviral Antibiotics" JOURNAL OF ANTIBIOTICS., Bd. 43, Nr. 3, März 1990, TOKYO JP, Seiten 267-280, XP002049159 in der Anmeldung erwähnt
- H. ITOKAWA ET AL.: "Structural and Conformational Studies of [Ile7] and [Leu7] Surfactings from Bacillus subtilis natto" CHEMICAL AND PHARMACEUTICAL BULLETIN., Bd. 42, Nr. 3, März 1994, TOKYO JP, Seiten 604-607, XP002049160 in der Anmeldung erwähnt
- B. HOROWITZ ET AL.: "Inactivation of Lipid-Enveloped Viruses in Labile Blood Derivatives by Unsaturated Fatty Acids" VOX SANGUINIS, Bd. 54, Nr. 1, 1988, Seiten 14-20, XP002049158

## Beschreibung

Die Erfindung betrifft ein äußerst effizientes Verfahren zur Inaktivierung von lipidumhüllten Viren, wie z.B. Herpes- oder Retroviren, in biologischen oder biotechnologischen - insbesondere pharmazeutischen - Produkten und in Zellkulturen, indem ein zyklisches Lipopeptid oder ein Gemisch von Lipopeptiden oder deren Salze oder Ester in bestimmten Konzentrationen zugesetzt werden. Es hat sich gezeigt, daß Lipopeptide ein überraschend hohes Inaktivierungspotential für lipidumhüllte Viren aufweisen und daneben den Vorteil einer sehr geringen in vivo-Toxizität bieten, wodurch der Schritt der Entfernung des Inaktivierungsagenzes aus den pharmazeutischen Produkten oder aus der Zellkultur entfallen kann. Gegenstand der Erfindung sind auch neue antivirale Lipopeptide, die zu den Surfactinen gehöhren.

Spätestens mit der AIDS-Epidemie ist die Erkenntnis in das Bewußtsein der breiten Öffentlichkeit gerückt, daß nicht nur HI-Viren sondern eine Vielzahl humanpathogener Erreger z.B. durch Bluttransfusionen, Pharmazeutika, Transplantationen usw. übertragbar sind. Generell muß heutzutage jedes aus biologischem Material hergestellte oder damit in Berührung gekommene Pharmakon als potentiell mikrobiell bzw. viral kontaminiert eingestuft und die Infektionssicherheit nachgewiesen werden. Durch die Entwicklung molekularbiologischer Methoden zur Herstellung von Pharmaka ist das Infektionsrisiko durch unterschiedliche mikrobielle Verunreinigungen nochmals gestiegen. Bei der biotechnologischen Herstellung von Pharmazeutika werden häufig animale oder humane Zellinien verwendet. Besonders bei diesen Zellen können Virusinfektionen durch endogene Viren, latente Virusinfektionen oder Kontaminationen nicht vollkommen ausgeschlossen werden. Die Infektionssicherheit von biotechnologischen Pharmazeutika, z.B. Impfstoffen, monoklonalen Antikörpern, Hormonen oder rekombinanten Proteinen erfordert daher die Entfernung jeglicher infektiöser, nicht ertaünschter Partikel, was grundsätzlich mit beträchtlichen Verlusten an Forschungszeit und -mitteln bzw. Produktivität verbunden sein kann. Die Virussicherheit von Blut und Blutprodukten kann nur durch Prüfung und Selektion der Blutspenden in Kombination mit der Evaluierung und prophylaktischen Anwendung von wirkungsvollen und zuverlässigen Virusinaktivierungs- und Viruseliminierungsverfahren gewährleistet werden.

Zur Inaktivierung und Eliminierung von Viren aus pharmazeutischen Produkten werden verschiedenartige Methoden einzeln oder in Kombination genutzt. Bei strukturell einfachen und stabilen Produkten kommen chromatographische Methoden, pH-"shift", Extraktion und Fraktionierung mit verschiedenen organischen Lösungsmitteln, Salzpräzipitation, Hitzebehandlung und Filtrationstechniken zum Einsatz [Rabenau & Doerr (1990) Die Infektionssicherheit biotechnologischer Pharmazeutika aus virologischer Sicht, S. 58, GIT VERLAG GmbH, Darmstadt]. Bei empfindlichen oder komplexen biologischen Materialien werden häufig antiviral wirkende Substanzen eingesetzt. Folgende Methoden kommen u.a. zum Einsatz:
- kombinierte Anwendung von Lösungsmitteln (z.B. Etherextraktion) und synthetischen Detergenzien (z.B. Triton X-100) [B. Horowitz et al. (1985) Transfusion 25 516-522],
- Einsatz von β-Propiolacton in Kombination mit UV-Licht sowie Methylenblau in Kombination mit einer Photoaktivierung [W. Stephan (1989) S. 122-127 in: J.-J. Morgenthaler (Ed.); Virus inactivation in plasma products.; Curr. Stud. Hematol. Blood Transfus.; No. 56; Karger Basel],
- Pasteurisierung von flüssigem Material [T. Nowak (1992) Biologicals 20 83-85],
- Erhitzung von lyophylisiertem Material [D. Piszkiewicz *et al.* (1989) S. 44-54 in: J.-J. Morgenthaler (Ed.); Virus Inactivation in plasma products.; Curr. Stud. Hematol. Blood Transfus.; No. 56; Karger Basel],
- Bestrahlung mit Gammastrahlen (z.B. Cobalt-60) [B. Horowitz *et al.* (1988) Transfusion 25 523-527].

In der Literatur werden eine Reihe von Virusinaktivierungsverfahren für Blutprodukte, insbesondere von humanem Blutplasma, beschrieben. So offenbart A.M. Prince in US 4.591.505 ein Inaktivierungsverfahren für Hepatitis B-Viren, bei dem Alkohol und als virusinaktivierendes Agenz entweder ein nichtionisches Detergenz oder Ether oder ein Gemisch aus beiden den Blutprodukten zugesetzt wird. Als nichtionische Detergenzien kommen Polyoxyethylenderivate oder Sulfobetaine zur Anwendung.

B. Horowitz beschreibt in US 4.841.023 und in Vox-Sang. 54: 14 - 20 (1988), S. Karger AG, Basel, die Inaktivierung von lipidenthaltenden Viren in Blutprodukten durch Fettsäuren und in US 4.613.501 durch C₁₋C₄-Alkyl-Oleinsäure.

Ein Verfahren zur Herabsetzung unerwünschter Aktivitäten wie Pyrogenität, Hepatitis-Infektiösität und Aggregation in biologischen und pharmazeutischen Produkten, insbesondere auch Blutprodukten, durch Behandlung mit nicht-denaturierenden Amphiphilen, wie nicht-ionischen Tensiden (z.B. Tween 80) wird von E. Shanbrom in EP 0 050 061 offenbart.

Die Inaktivierung und Eliminierung von Viren aus Zellkulturen erfolgt durch die Anwendung von antiviralen Substanzen, die in der Regel die Virusreplikation hemmen.

Keines der bisher eingesetzten Inaktivierungsverfahren kann mit Sicherheit alle Viren inaktivieren oder eliminieren, die in biologischem Material vorkommen können.
Verfahren wie die Pasteurisierung oder die Hitzebehandlung benötigen in der Regel die Anwendung von Stabilisatoren; zudem besteht das Problem der Denaturierung von Proteinen. Die Anwendung von Lösungsmitteln und synthetischen Tensiden, angesehen als geeignet zur Inaktivierung lipidumhüllter Viren, konnte bisher durch schwankende Ergebisse in den Inaktivierungskinetiken oder durch fehlende systematische Untersuchungen bedingt durch die hohe Toxizität der Substanzen in Zellkulturen nicht als vollständig sicher beurteilt werden. Bei einer Vielzahl von biotechnologischen Produkten sind aufgrund der Struktur oder der Stabilität aufwendige Reinigungen oder eine Inaktivierung mit produktschädigenden oder zytotoxischen antiviralen Substanzen wie Lösungsmitteln nicht möglich.

Aufgabe der Erfindung war es deshalb, ein schonendes Verfahren zur Inaktivierung von lipidumhüllten Viren in biologischen oder biotechnologischen Produkten und in Zellkulturen zur Verfügung zu stellen, mit dem diese Produkte oder Zellkulturen sehr schnell und effektiv virusfrei gemacht werden können, ohne daß die Produkte denaturieren oder die Zellkulturen in ihrer Produktivität beeinträchtigt werden. Das Verfahren soll auch die Behandlung temperaturlabiler Produkte ermöglichen und nicht mit Substanzen arbeiten, die in vivo toxisch sind.

Die erfindungsgemäße Aufgabe wird durch den Einsatz von zyklischen β-Hydroxyfettsäure- und β-Aminofettsäurehaltigen Peptiden (Lipopeptiden) gelöst. Es hat sich gezeigt, daß die Lipopeptide ein überraschend hohes Inaktivierungspotential für lipidumhüllte Viren aufweisen und damit hervorragend zur Lösung der erfindungsgemäßen Aufgabe eingesetzt werden können. Lipopeptide erwiesen sich teilweise wesentlich wirksamer als die bisher zur Inaktivierung von Viren eingesetzten synthetischen Tenside. Daneben sind sie leicht biologisch abbaubar und besitzen eine wesentlich geringere in vivo-Toxizität als die synthetischen Tenside. Gegenüber herkömmlichen antiviralen Substanzen haben die erfindungsgemäß eingesetzten Lipopeptide den Vorteil, daß sie thermisch stabil und gut wasserlöslich sind.
Aus der Literatur ist bekannt, daß zwei [Ile⁷] und [Leu⁷] Surfactine, die zu den Lipopeptiden gehören, moderate Anti-HIV-1-Aktivität zeigen (H. Itokawa et al., Chem. Pharm. Bull. 42, 604 - 607 (1994)). Pumilacidine werden von N. Naruse et al. in Journal of Antibiotics, Japan XLIII 267 - 280 (1989) als antiviral wirksam gegen das Herpes-simplex-Virus (HSV-1) beschrieben. In keiner der beiden Arbeiten finden sich jedoch Hinweise auf das beträchtliche Inkaktivierungspotential dieser Substanzen, das eine umfassende Inaktivierung von lipidumhüllten Viren mit geringen Konzentrationen in kürzester Zeit erlaubt.

So ist das erfindungsgemäße Inaktivierungsverfahren dadurch gekennzeichnet, daß den biologischen oder biotechnologischen Produkten ein Lipopeptid oder dessen Salz oder Ester oder ein Gemisch von Lipopeptiden oder deren Salze oder Ester in einer Gesamtkonzentration von 1 - 100 µM, vorzugsweise 1 - 80 µM, zugesetzt werden und die Inaktivierung bei Raumtemperatur innerhalb von 30 min bis maximal 2h durchgeführt führt, wobei bereits nach 30 min ca. 99% der Viren inaktiviert sind. Bedingt durch die sehr geringe in vivo-Toxizität der erfindungsgemäß eingesetzten Lipopeptide ist es auch möglich, diese Inaktivierungssubstanzen in den o.g. Konzentrationen in den pharmazeutischen Produkten zu belassen. Nach erfolgter Inaktivierung können die eingesetzten Lipopeptide jedoch auch durch "reversed phase"-HPLC an C₁₈-Säulen oder Adsorptionschromatographie an Silicagel-Säulen aus den Produkten entfernt werden.

Da die erfindungsgemäß eingesetzten Lipopeptide thermisch stabil sind, kann das Inaktivierungsverfahren in Abhängigkeit von der thermischen Stabilität der zu behandelnden Produkte auch bei höheren Temperaturen durchgeführt werden, vorzugsweise bei 30 - 60°C. Es hat sich gezeigt, daß die Inaktivierungsleistung linear von der Temperatur abhängig ist. So bewirkt eine Steigerung der Temperatur um 10°C bereits einen Anstieg der Inaktivierungsrate um den Faktor 2,4, so daß die Virusinaktivierung bei 30 - 60°C mit den genannten Konzentrationen schon innerhalb von 5 - 30 min möglich ist. Es ist gene-rell auch möglich, bei tieferen Temperaturen bis zu 0°C die Viren zu inaktivieren, wobei dies jedoch je nach Spezies länger als 2 Stunden dauern kann.

Erfindungsgemäß ist die Virusinaktivierung in Zellkulturen dadurch gekennzeichnet, daß dem serumfreien Kulturmedium ein Lipopeptid oder dessen Salz oder Ester oder ein Gemisch von Lipopeptiden oder deren Salze oder Ester in einer Gesamtkonzentration von 1 - 65µM, vorzugsweise von 1 - 50µM zugesetzt werden. Wird mit serumhaltigem Kulturmedium gearbeitet, das bis zu 5 Vol.-% Serum, z.B. FKS, beinhaltet, so beträgt die zur vollständigen Inaktivierung notwendige Lipopeptidkonzentration von 10 - 100µM, vorzugsweise von 30 - 90µM.

Das erfindungsgemäße Inaktivierungsverfahren kann in einem breiten pH-Bereich von 4 - 9, vorzugsweise von 5,5 - 8, durchgeführt werden.

Im Inaktivierungverfahren der Erfindung können natürlich vorkommende, chemisch synthetisierte, gentechnisch hergestellte und gentechnisch modifizierten zyklischen Lipopeptide eingesetzt werden.

Die erfindungsgemäß eingesetzten zyklischen Lipopeptide können nach bereits beschriebenen und dem Fachmann bekannten Verfahren leicht hergestellt werden. Zahlreiche Lipopeptide werden u.a. vom Mikroorganismus *Bacillus subtilis in vivo* gebildet und in das umgebende Medium in hohen Konzentrationen sekretiert, aus dem sie dann isoliert werden können.

Bei den Viren, die mit den erfindungsgemäßen Verfahren inaktiviert werden können, handelt es sich vor allem um Herpes-Viren, vorzugsweise HSV-1, HSV-2, BHV-1, SHV-1, Immundefizienz-Viren, vorzugsweise HIV-1, HIV-2, SIV_{agm}, das vesikuläre Stomatitis-Virus (VSV) und das Semliki-Forest-Virus (SFV). Aber auch andere lipidumhüllte Viren lassen sich erfindungsgemäß effektiv inaktivieren.

Erfindungsgemäß bevorzugt werden zur Inaktivierung die Lipoheptapeptide der allgemeinen Formel I, die auch als Surfactine bezeichnet werden, deren Salze, Ester oder deren Gemische eingesetzt 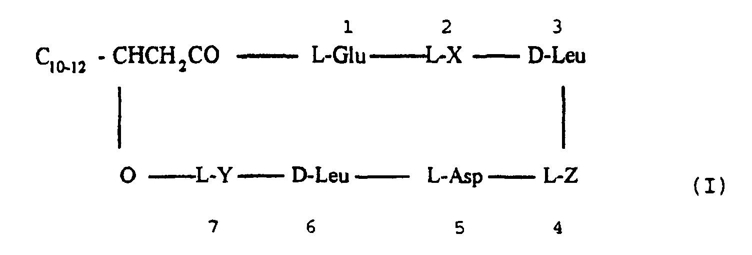 wobei in der Formel I X und Y für die Aminosäuren Leu, Ile oder Val, Z für die Aminosäuren Val oder Ala steht und C₁₀₋₁₂ eine lineare oder verzweigte, gesättigte Alkylkette bedeutet. Erfindungsgemäß handelt es sich bei den Surfactinen der allgemeinen Formel I mit X als Val oder Ile und deren Ester um neue Verbindungen, die ebenfalls Gegenstand der vorliegenden Erfindung sind.

Es hat sich gezeigt, daß zum Beispiel das vom Stamm Bacillus subtilis ATCC 21332 und das vom produktiveren Stamen Bacillus subtilis OKB 105 produzierte Surfactingemisch sehr gut zur erfindungsgemäßen Inaktivierung von Viren geeignet ist. Das Surfactin der Bacillus subtilis-Stämme ist ein Gemisch aus Isoformen, daß heißt aus Verbindungen der allgemeinen Formel I, die sich untereinander in der Kettenlänge der Fettsäure, in der Verzweigung der Fettsäure und in den Aminosäuren X, Y und Z, wie oben dargestellt, unterscheiden.
Auch Einzelverbindungen der allgemeinen Formel I, so zum Beispiel Surfactinisoformen mit einem Fettsäurerest von C₁₄-Alkyl oder C₁₅-Alkyl (daß heißt C₁₁- oder C₁₂ Alkyl in der allgemeinen Formel I), die zum Beispiel aus dem durch Fermentation der genannten Stämme erhaltenen Surfactingemisch isoliert oder auch chemisch synthetisiert werden können, zeigen ein hohes Inaktivierungspotential. So wurde festgestellt, daß zum Beispiel Surfactine der allgemeinen Formel I mit C₁₅-Alkyl als Fettsäurerest Vesikulärstomatitisvirus (VSV) noch schneller inaktivieren als das Surfactingemisch. Surfactine mit C₁₄-Alkyl als Fettsäurerest haben sich als effektive Verbindungen zur Inaktivierung von Schweineherpesvirus (SHV-1) erwiesen.

Erfindungsgemäß eingesetzt werden können auch Pumilacidine der allgemeinen Formel I als Einzelkomponenten oder im Gemisch, wie sie zum Beispiel in Journal of Antibiotics, Japan XLIII 267-280 (1989), Seite 267-280 beschrieben sind sowie deren Salze oder Ester.

In einer spezifischen Ausführungsform werden im erfindungsgemäßen Verfahren Verbindungen der allgemeinen Formel I eingesetzt, deren Aminosäuren Glu und/oder Asp verestert sind. Die Monoester der Verbindungen der allgemeinen Formel I, in denen nur eine der genannten Aminosäuren verestert ist, zeigen ganz spezifische Effekte. So hat sich gezeigt, daß zum Beispiel C₁₄-Alkyl-Monoester der allgemeinen Formel I das Schweineherpes-Virus in 20 Minuten bei einer Konzentration von 40 µM um den Faktor >10⁴ inaktivieren. C₁₅-Alkyl-Monoester der allgemeinen Formel I waren ebenfalls in der Lage, Semliki-Forest-Virus (SFV) in 20 Minuten bei einer Konzentration von 40 µM um den Faktor >10⁴ zu inaktivieren. Beispielhaft sei die Inaktivierung von SFV mit C₁₄ bzw. C₁₅-Alkyl-Monoestern in nachfolgender Tabelle 1 dargestellt:

**Tabelle 1:**

| Inaktivierung von SFV | | | | | | |
|---|---|---|---|---|---|---|
| Lipopeptid | Inkubationsdauer (min) | | | | | |
| | 0 | 1 | 5 | 10 | 15 | 20 |
| | Titer (TCID₅₀/ml) | | | | | |
| Monoester C₁₄ | 2,4 * 10⁴ | 1,7 * 10³ | 98,0 | 30,0 | 30,0 | 30,0 |
| Monoester C₁₅ | 2,5 *10⁵ | 173,0 | 99,0 | 25,0 | 10,0 | 6,5 |

Im Sinne der Erfindung bedeuten biologische Produkte aus Säugern isolierte Produkte, wie z.B. Blutprodukte und aus Blut isolierte Produkte wie z.B. Impfstoffe und Plasmaderivate. Unter biotechnologischen pharmazeutischen Produkten werden biotechnologisch hergestellte Wirkstoffe wie z.B. Humanproteine (hGH, TNF, t-PA, EPO) oder Gerinnungsfaktoren (z.B. Faktor VIII) verstanden, wobei die Erfindung jedoch nicht auf die genannten Produkte aus Zellkulturen beschränkt ist.

Nachfolgend wird die Erfindung anhand von Ausführungsbeispielen näher erläutert.

### Beispiel 1

### Herstellung der Surfactine der allgemeinen Formel I

Das erfindungsgemäß eingesetzte Surfactingemisch, das die Verbindungen der allgemeinen Formel I enthält, wird durch Kultivierung von Bacillus subtilis-Stämmen, insbesondere von Bacillus subtilis ATCC 21332 bzw. Bacillus subtilis OKB 105 wie in Biochemical and Biophysical Research Communications, Vol. 177, Nr. 3 (1991), Seite 998-1005 beschrieben, hergestellt und gereinigt.

### Beispiel 2:

### Isolierung der Isoformen aus dem gemäß Beispiel 1 hergestellten Surfactingemisch

Die Isolierung der Einzelverbindungen der allgemeinen Formel I erfolgte durch präparative RP HPLC an EnCa Pharm 100 RP 18-TS (5 µm, 250 * 16 mm) mit Hilfe eines LKB HPLC-Systems. Die Surfactinisoformen wurden mit Hilfe von Acetonitrilgradienten eluiert Lösungsmittel A enthielt 40% Acetonitril und 60% 10mM NH₄OAC, pH=6,9 (v/v). Lösungsmittel B war 100% Acetonitril. Die Isoformen wurden nach Lyophilisierung als weißes Pulver erhalten and NMR-spektroskopisch und MS-spektrometrisch bestimmt. Tabelle 2 zeigt die isolierten Verbindungen.

**Tabelle 2:**

| Isolierte Isoformen | | | |
|---|---|---|---|
| (In der nachfolgenden Tabelle ist die Aminosäure (AS) in Position 4 Valin und die AS2 ist Leucin, falls nicht anders ausgewiesen) | | | |

| N° der Fraktion | Aminosäuren | Molekulare Masse in Dalton | Fettsäure-Kettenlänge |
|---|---|---|---|
| 1 | [Leu7]- | 1008 | C₁₃ |
| 2 | [Val7]- | 994 | C₁₃ |
| 3 | [Ile2, Val7] - | 994 | C₁₃ |
| 4 | [Leu7] - | 1022 | C₁₄ |
| | [Ile7] - | 1008 | C₁₃ |
| 5 | [Val7] - | 1008 | C₁₄ |
| 6 | [Ile2, Val7]- | 1008 | C₁₄ |
| 7 | [Leu7] - | 1036 | C₁₅ |
| 8 | [Ile7] - | 1022 | C₁₄ |
| 9 | [Val7] - | 1022 | C₁₅ |
| 10 | [Ile2, Val7] - | 1022 | C₁₅ |
| 11 | [Ile7] - | 1036 | C₁₅ |
| 12 | [Ile2, Ile7] - | 1036 | C₁₅ |

Bei den Fraktionen 3, 6, 10 und 12 handelt es sich um neue Verbindungen, [Ile2, Val7]- Surfactine und [Ile2, Ile7] - Surfactin, die bisher noch nicht beschrieben wurden. Nachfolgend sind die ¹H-NMR- Daten der Verbindungen in den Fraktionen 10 und 12 bei 30°C in Pyridin-d₅ dargestellt:

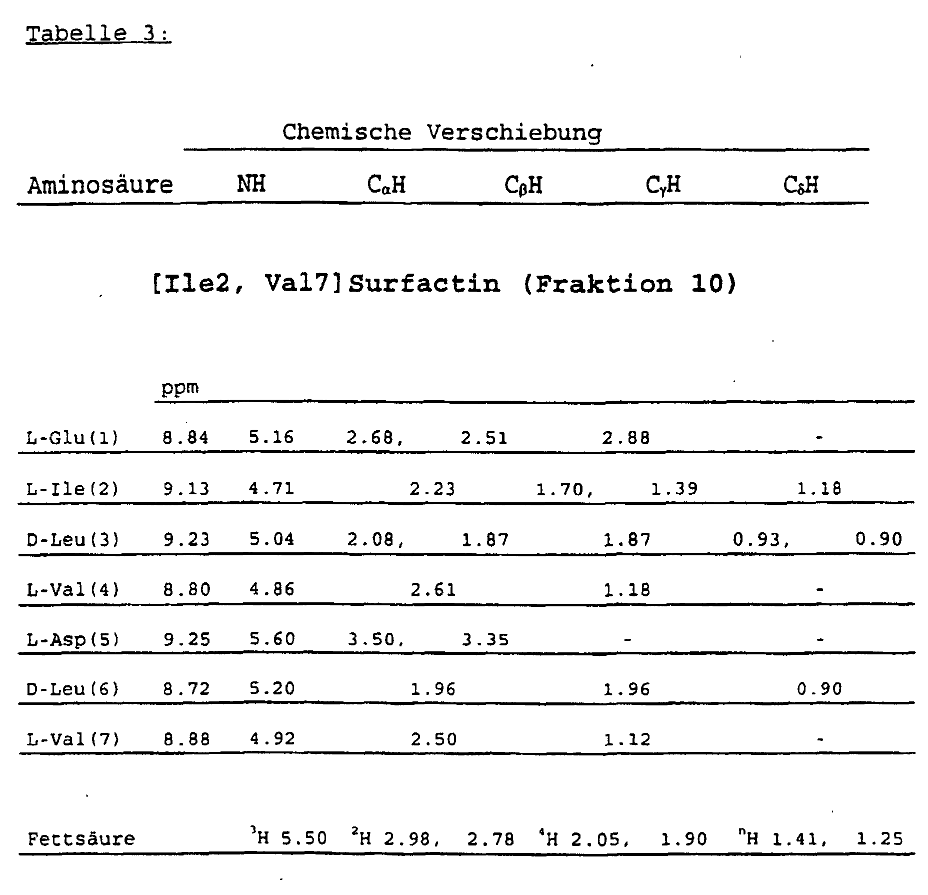

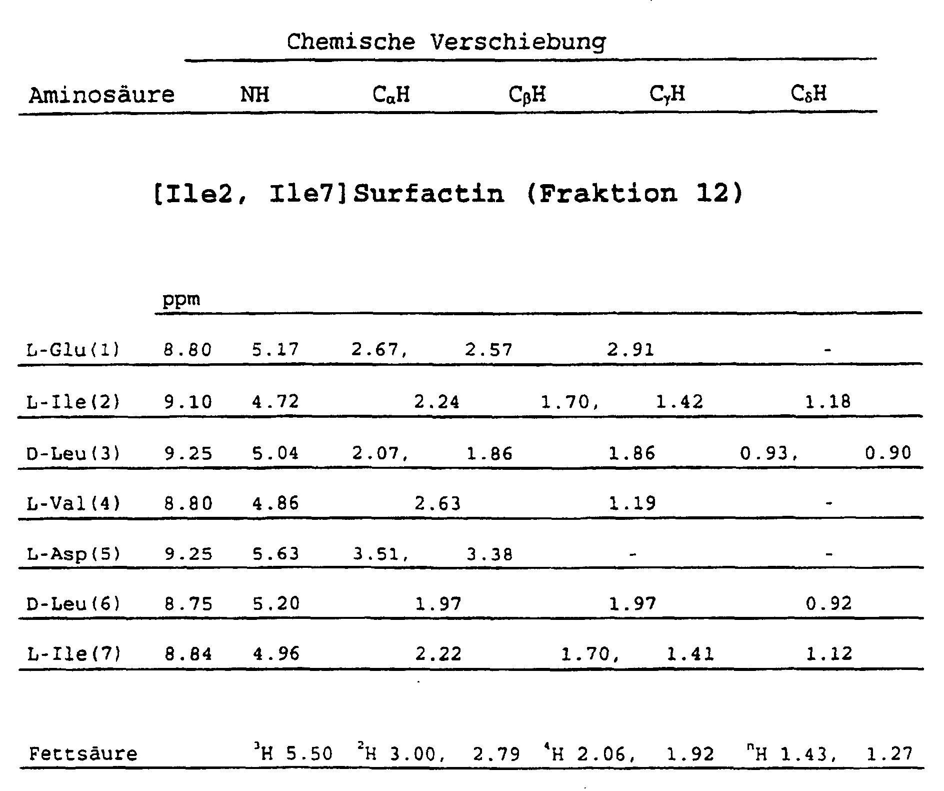

### Beispiel 3:

### Herstellung von Monomethylestern der Verbindungen der allgemeinen Formel I

Es wurde eine Veresterung des in Beispiel 1 erhaltenen Surfactingemisches mit Methanol/HCl durchgeführt und die einzelnen Monomethylester isoliert. Zur Herstellung der Monomethylester wurden 100 mg Surfactingemisch mit 50 ml Methanol und 5 ml HCl (pH=0,3) für 24 Stunden inkubiert.

Die Mischung wurde bei Reaumtemperatur gerührt, das Lösungsmittel wurde im Vakuum entfernt. Das erhaltene Verhältnis von Surfactin/Surfactin-Monomethylester/ Surfactin-Dimethylester betrug ca. 0,1:2:1. Die Monomethylester wurden mittels präparativer RP HPLC mit derselben Säule, wie in Beispiel 2 beschrieben, isoliert. Der Acetonitrilgradient liegt hier zwischen 35 und 50% B. Tabelle 4 zeigt die isolierten Surfactin-Moomethylester.

**Tabelle 4**

| (In der nachfolgenden Tabelle ist die Aminosäure (AS) in Position 4 Valin und die AS2 ist Leucin, falls nicht anders ausgewiesen) | | | |
|---|---|---|---|
| 14 | [Val7] - | 1008 | C₁₃ |
| 15 | [Leu7] - | 1022 | C₁₃ |
| | [Val7] - | 1008 | C₁₃ |
| 16 | [Leu7] - | 1022 | C₁₃ |
| | [Ile2, Val7] - | 1008 | C₁₃ |
| 18 | [Ile7] - | 1022 | C₁₃ |
| | [Val2, Val7] - | 1008 | C₁₄ |
| 19 | [] Ile7- | 1022 | C₁₃ |
| 20 | [Val7]- | 1022 | C₁₄ |
| 21 | [Leu7] - | 1036 | C₁₄ |
| 22 | [Val7] - | 1022 | C₁₄ |
| | [Ile2, Val7]- | 1022 | C₁₄ |
| 23 | [Leu7] - | 1036 | C₁₄ |
| 24 | [Ile7] - | 1036 | C₁₄ |
| 25 | [Val7] - | 1036 | C₁₅ |
| | [Ile7] - | 1036 | C₁₄ |
| 26 | [Leu7] - | 1050 | C₁₅ |
| | [Val7] - | 1036 | C₁₅ |
| 27 | [Leu7] - | 1050 | C₁₅ |
| | [Ile2, Val7] - | 1036 | C₁₅ |
| 28 | [Ile7] - | 1050 | C₁₅ |
| 29 | [Ile7] - | 1050 | C₁₅ |
| 30 | [Ile2, Ile7] - | 1050 | C₁₅ |

### Beispiel 4:

### Herstellung von Dimethylestern der Verbindungen der allgemeinen Formel I

Die Veresterung von 100 mg des Surfactingemisches aus Beispiel 1 wurde mit 5 ml 37% HCl und 50 ml Methanol in 48 Stunden durchgeführt. Die Diester waren die Hauptprodukte und wurden wie in Beispiel 3 beschrieben isoliert. Tabelle 5 zeigt die isolierten Surfactin-Dimethylester.

**Tabelle 5**

| | | | |
|---|---|---|---|
| 34 | [Val7] - | 1022 | C₁₃ |
| 35 | [Leu7] - | 1036 | C₁₃ |
| 36 | [Ile7] - | 1036 | C₁₃ |
| 37 | [Val2, Val7]- | 1022 | C₁₄ |
| 38 | [Val7] - | 1036 | C₁₄ |
| 39 | [Leu7] - | 1050 | C₁₄ |
| 40 | [Ile7] - | 1050 | C₁₄ |
| | [Ile2, Val7] - | 1036 | C₁₄ |
| 41 | (Val7)- | 1050 | C₁₅ |
| 42 | [Leu7] - | 1064 | C₁₅ |
| 43 | [Ile7] - | 1064 | C₁₅ |
| | [Ile2, Val7] - | 1050 | C₁₅ |
| 44 | [Ile2, Ile7] - | 1064 | C₁₅ |

Für den Diester der Fraktion 37 werden nachfolgend beispielhaft die ¹H-NMR-Daten aufgezeigt, da es sich bei diesem Ester und der dem Ester zugrunde liegenden Isoform des Surfactins um eine neue Verbindung mit der Aminosäure Valin in Position 2 handelt.

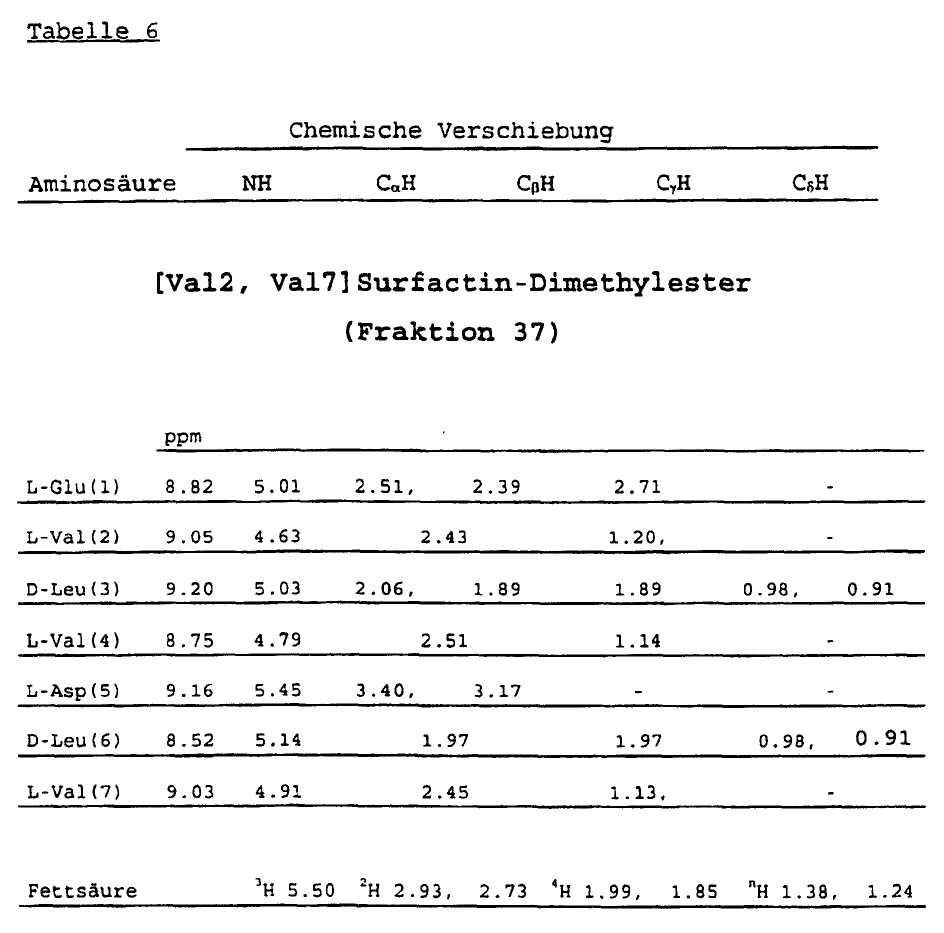

### Beispiel 5

### Bestimmung der antiviralen Aktivität

Zur Bestimmung der antiviralen Aktivität von zyklischen Lipopeptiden wurden Herpes-Viren mit dem Surfactingemisch aus Bacillus subtilis (im weiteren immer als Surfactin bezeichnet) behandelt und zeitabhängig die Anzahl der infektiösen Viruspartikel durch Aussaat auf frische Wirtszellen (Endstufentitration) bestimmt.
1. Herpes simplex-Virus Typ 1 (HSV-1) wurde in 50 ml Dulbecco's modifiziertem Eagle's Medium (ICN) mit 5% (v/v) über 30 min bei 56°C inaktiviertem fötalem Kälberserum (GIBCO) und 80 µM Surfactin, sterilfiltriert über Nalgene Spritzenvorfilter mit einer Porenweite von 0,1 µm, aufgenommen. Der Anfangstiter betrug 5x10⁵ ID₅₀/ml. Der pH-Wert des Inaktivierungsansatzes betrug während der Versuchsdauer durch Zugabe von 1 N HCl konstant pH 7,8 und die Temperatur 22°C.
2. Aus dem permanent gerührten Inaktivierungsansatz wurden zu den Zeitpunkten 5, 10, 15, 30, 45, 60, 90, 120 und 180 Minuten Aliquots entnommen. Von diesen Aliquots wurde aus einer 1:10 Vorverdünnung heraus eine Verdünnungsreihe zur Basis 3 erstellt. Die Verdünnungen wurden auf eine 96-Loch Flachboden-Mikrotiterplatte (Nunc) in 8 parallele Reihen mit jeweils 100 µl einer jeden Verdünnungsstufe überführt. Die 96 Löcher der Mikrotiterplatte waren zuvor mit jeweils 100 µl einer Vero-Zell-Suspension mit einer Zelldichte von 1,5x10⁵ Zellen/ml beschickt worden.
3. Die Platten wurden 6 Tage bei 37°C und 5 Vol.-% CO₂ bebrütet. Die Zellen in den Kontrollansätzen ohne Virusverdünnung waren zu hohen Dichten herangewachsen. Die Zellkulturen der Mikrotiterplatte wurden lichtmikroskopisch untersucht. Jede Kultur mit Anzeichen eines zytopathogenen Effektes wurden als infiziert gewertet.
4. Die Titer wurden als 50%ige infektiöse Dosis (ID₅₀) nach der Methode von Spearman und Kärber [in: Biometrie. Grundzüge biologisch-medizinischer Statistik. (1974) Ed. L. Cavalli-Sforza. Gustav Fischer-Verlag Stuttgart S. 171-173] berechnet und auf 1 ml des Inaktivierungsansatzes bezogen.

Innerhalb von 15 Minuten reduzierte sich der Anfangtiter von 5,1x10⁵ ID₅₀ auf eine Restinfektiösität von 20 ID₅₀. Nach 30 Minuten Inkubationszeit betrug der Titer an infektiösem HSV-1 7 ID₅₀. Nach 60 Minuten konnten keine infektiösen Partikel mehr gefunden werden. Surfactin wirkt stark viruzid auf das Herpes simplex-Virus und ermöglicht eine Inaktivierung von 5 log₁₀ HSV-1-Partikeln in Serum-haltigem Kulturmedium in weniger als 60 Minuten. Damit nahm die Anzahl der umhüllten infektiösen Viruspartikel bei der Anwendung von Surfactin mit einer wesentlich höheren Geschwindigkeit als bei bisher angewandten Inaktivierungsverfahren ab.

### Beispiel 6

### Bestimmung des Wirkungsspektrums

Zur Bestimmung des antiviralen Wirkungsspektrums von zyklischen Lipopeptiden wurden Viren, die u.a. als besonders resistent gegen physikalische und chemische Inaktivierungsmethoden angesehen werden, mit Surfactin behandelt und zeitabhängig die verbliebene Infektiösität der Viruspartikel bestimmt.
1. In 50 ml Dulbecco's modifiziertem Eagle's Medium (ICN) mit 5% (v/v) frisch über 30 min bei 56°C inaktiviertem fötalem Kälberserum (GIBCO) und 80 µM Surfactin, sterilfiltriert über Nalgene Spritzenvorfilter mit einer Porenweite von 0,1 µm, wurden die in Tabelle 1 aufgeführten membranumhüllten Viren aufgenommen. Die eingesetzten Anfangstiter sind in Tabelle 1 gezeigt. Der pH-Wert des Inaktivierungsansatzes betrug während der Versuchsdauer durch Zugabe von 1 N HCl konstant pH 7,8 und die Temperatur 22°C.

**Tabelle 1:**

| In den Inaktivierungsversuchen verwendete Virus/Zell-Systeme | | | |
|---|---|---|---|
| Virus | Anfangstiter [ID₅₀/ml] | Wirtszellinie | Modellvirus für |
| Schweine-Herpes-Virus (SHV-1) | 1,6x10⁵ | Nerzlungen-Zellen | humane Herpesviren |
| Bovines-Herpes-Virus (BHV-1) | 4,3x10⁵ | Rindernieren-Zellen | humane Herpesviren |
| Herpes-simplex-Virus Typ2 (HSV-2) | 4,2x10⁴ | Affennieren-Zellen | |
| Immundefizienzvirus von Affen (SIV_{agm}) | 1,6x10⁵ | humane T-Helfer- Zellen | humane Immundefizienzviren |
| Vesikuläres Stomatitis Virus (VSV) | 9,5x10⁶ | Babyhamster-Fibroblasten | oft verwendetes Modellvirus |
| Semliki-Forest-Virus (SFV) | 7,0x10⁷ | Babyhamster-Fibroblasten | westliche/östliche Pferdeenzephalitis |

2. Aus dem permanent gerührten Inaktivierungsansatz wurden zu den Zeitpunkten 5, 10, 15, 30, 45, 60, 90, 120, 180 und 360 Minuten Aliquots entnommen. Von diesen Aliquots wurde aus einer 1 : 10 Vorverdünnung heraus eine Verdünnungsreihe zur Basis 3 erstellt. Die Verdünnungen wurden auf eine 96-Loch Flachboden-Mikrotiterplatte (Nunc) in 8 parallele Reihen mit jeweils 100 µl einer jeden Verdünnungsstufe überführt. Die 96 Löcher der Mikrotiterplatte waren zuvor mit jeweils 100 µl der entsprechenden Wirtszell-Suspension (Tabelle 1) mit Zelldichten von ca. 1,5x10⁵ bis 5x10⁴ Zellen/ml, entsprechend der Proliferationsrate der Wirtszelle, beschickt worden.
3. Die Platten wurden je nach Zellinie 6 bis 18 Tage bei 37°C und 5 Vol.-% CO₂ bebrütet. Die Zellen in den Kontrollansäzen ohne Virusverdünnung waren zu hohen Dichten herangewachsen. Die Zellkulturen der Mikrotiterplatte wurden lichtmikroskopisch untersucht. Jede Kultur mit Anzeichen eines zytopathogenen Effektes wurden als infiziert gewertet. Der zytopathogene Effekt von SIV_{agm} auf die humane T-Helfer-Zellinie MOLT 4/8 wurde aufgrund der unzureichenden lichtmikroskopischen Auswertbarkeit entsprechend der Beschreibung des Zellproliferationabhängigen Tests nach Mosman [J. Immunol. Meth. 65 55-63 1983] mit dem Farbstoff MTT durchgeführt.
4. Die Titer wurden entsprechend der Beschreibung in Beispiel 1, Abschnitt 5, berechnet.

Alle aufgeführten umhüllten Viren ließen sich durch die Anwendung von Surfactin in Serum-haltigem Medium inaktivieren. Innerhalb von 120 Minuten waren weniger als 0,02% der eingesetzten Infektiösität von SHV-1, BHV-1 und HSV-2 nachweisbar. Dieser Anteil an Restinfektiösität wurde bei den Viren VSV und SIV_{agm} bereits nach einer Inkubationszeit von 60 Minuten erreicht. Das Lipopeptid Surfactin wirkt direkt auf eine Vielzahl von lipidumhüllten Viren mit hoher Inaktivierungsgeschwindigkeit.

### Beispiel 7

### Einfluß der Lipopeptid-Konzentration

Der Einfluß der Konzentration an zyklischem Lipopeptid auf die Inaktivierungsleistung kann durch die Bestimmung der Inaktivierungsrate (Abnahme der Virusinfektiösität pro Inaktivierungsdauer) quantifiziert werden.
1. Schweine-Herpes-Virus (SHV-1) wurde in 25 ml Dulbecco's modifiziertem Eagle's Medium (ICN) mit 5% (v/v) über 30 min bei 56°C inaktiviertem fötalem Kälberserum (GIBCO) und Surfactinkonzentrationen von jeweils 0, 10, 20, 30, 40, 50, 60, 70, 75, 80, 90 und 100 µM aufgenommen. Das Surfactin wurde dazu in Kulturmedium gelöst und über Spritzenvorfilter (Nalgene, Porenweite 0,1 µm) sterilfiltriert. Der Anfangstiter betrug 2x10⁴ ID₅₀/ml. Die Temperatur im permanent gerührten Inaktivierungsansatz betrug 22°C und der pH-Wert 7,8.
2. Aus dem Inaktivierungsansatz wurden nach Zugabe von Surfactin zu den Zeitpunkten 2, 5, 10, 15 und 20 Minuten Aliquots entnommen und sofort 1:10 in Medium vorverdünnt.
3. Aus den Vorverdünnungen heraus wurde eine Verdünnungsreihe zur Basis 3 erstellt. Die Verdünnungen wurden auf eine 96-Loch Flachboden-Mikrotiterplatte (Nunc) in 8 parallele Reihen mit jeweils 100 µl einer jeden Verdünnungsstufe überführt. Die 96 Löcher der Mikrotiterplatte waren zuvor mit jeweils 100 µl einer Nerzlungen-Zell-Suspension mit einer Zelldichte von ca. 1,0x10⁵ Zellen/ml beschickt worden.
4. Die Inkubation der Zellkulturen in den Mikrotiterplatten, die Auswertung dieser Mikrotiterplatten sowie die Berechnung der Titer an verbliebenem infektiösem Virus zu den jeweiligen Abnahmezeiten wurde entsprechend der Beschreibung in Beispiel 1, Abschnitt 4 und 5, durchgeführt.

Die Inaktivierungsrate steigt exponentiell mit der Lipopeptid-Konzentrationen an. Im Bereich der Lipopeptidkonzentrationen von 10 bis 80 µM Surfactin stieg die Inaktivierungsrate von 0,19 auf 0,6 log₁₀ ID₅₀/10 min. Bei einer Surfactin- Konzentration von 100 µM wurden die eingesetzten infektiösen SHV-1-Partikel mit einer Rate von 1,4 log₁₀ ID₅₀/10 min inaktiviert. Das Lipopeptid Surfactin ermöglicht die Inaktivierung lipidumhüllter Viren in einem weiten Konzentrationsbereich. Die Lipopeptidkonzentration kann entsprechend den Produkt- und Verfahrenseigenschaften gewählt werden.

### Beispiel 8

### Virusinaktivierung bei verschiedenen Reaktionstemperaturen

Der Einfluß der Temperatur während der Inaktivierungreaktion auf die Virusinaktivierung kann durch die Bestimmung der Inaktivierungsrate (Abnahme der Virusinfektiösität pro Inaktivierungsdauer) quantifiziert werden.
1. Schweine-Herpes-Virus (SHV-1) wurde in 25 ml Dulbecco's modifiziertem Eagle's Medium (ICN) mit 5% (v/v) über 30 min bei 56°C inaktiviertem fötalem Kälberserum (GIBCO) und einer Surfactinkonzentrationen von 80 µM aufgenommen. Das Surfactin wurde in Kulturmedium gelöst und über Spritzenvorfilter (Nalgene, Porenweite 0,1 µm) sterilfiltriert. Der Anfangstiter betrug 1x10⁵ ID₅₀/ml. Der pH-Wert des permanent gerührten Inaktivierungsansatzes betrug während der Versuchsdauer durch Zugabe von 1 N HCl konstant pH 7,8. Die jeweiligen Inaktivierungsansätze wurden auf 5, 10, 15, 20, 25 und 30°C in einem Wasserbad [Grant, Modell W14 - ZD/CZ1] mit einer Genauigkeit von +/- 0,01°C eingestellt und permanent gerührt.
2. Aus dem Inaktivierungsansatz wurden nach Zugabe von Surfactin zu den Zeitpunkten 2, 5, 10, 15 und 20 Minuten Aliquots entnommen und sofort 1:10 in Medium vorverdünnt.
3. Aus den Vorverdünnung heraus wurde eine Verdünnungsreihe zur Basis 3 erstellt. Die Verdünnungen wurden auf eine 96-Loch Flachboden-Mikrotiterplatte (Nunc) in 8 parallele Reihen mit jeweils 100 µl einer jeden Verdünnungsstufe überführt. Die 96 Löcher der Mikrotiterplatte waren zuvor mit jeweils 100 µl einer Nerzlungen-Zell-Suspension mit einer Zelldichte von ca. 1,0x10⁵ Zellen/ml beschickt worden.
4. Die Inkubation der Zellkulturen in den Mikrotiterplatten, die Auswertung dieser Mikrotiterplatten sowie die Berechnung der Titer an verbliebenem infektiösem Virus zu den jeweiligen Abnahmezeiten wurde entsprechend der Beschreibung in Beispiel 1, Abschnitt 4 und 5, durchgeführt.

Im getesteten Temperaturbereich von 5 bis 30°C erfolgte eine deutliche Inaktivierung des SHV-1 im Serum-haltigen Kulturmedium. Die Inaktivierungsrate stieg linear von 0,05 für 5°C über 0,25 für 20°C nach 0,45 log₁₀ ID₅₀/min für 30°C an. Mit dem Lipopeptid Surfactin ist die Behandlung von temperaturlabilen Produkten zur Inaktivierung lipidumhüllter Viren wie auch eine zeitsparende schnelle Inaktivierung bei hohen Temperaturen möglich.

### Beispiel 9

### Bestimmung der Zytotoxizität von Surfactin auf adhärente Zellen

Die Bestimmung der zytotoxischen Wirkung der zyklischen Lipopeptide auf adhärente Zellen in Kultur erfolgte in Abwandlung des Kristallviolett-Tests von D.A. Flick und G.E. Gifford [J. Immunol. Meth. 68 167-175 1984].
1. Frisch trypsinierte Zellen der Linien ML (Nerz-Lungenzellen), CV1 (Nierenzellen der grünen Meerkatze), Hep₂ (humane Epithelzellen), CRFK (Katzen-Nierenzellen) und BHK21 (Hamster-Nierenzellen) wurden jeweils in Dulbecco's modifiziertem Eagle's Medium (ICN) mit 5% (v/v) frisch über 30 min bei 56°C inaktiviertem fötalem Kälberserum (GIBCO) aufgenommen und 100 µl Zellsuspension (ca. 1-2 x10⁵ Zellen/ml) in jeweils eine Vertiefung einer 96-Loch-Flachboden-Kulturplatte (Nunc) pipettiert. Die Kultur wurde 3 Stunden bei 37°C in Gegenwart 5 Vol.-% CO₂ bebrütet.
2. In Kulturmedium gelöstes Surfactin wurde sterilfiltriert (Nalgene Spritzenvorfilter, Porenweite 0,1 µm) und auf verschiedene Konzentrationen mit frischem Kulturmedium verdünnt. 50 µl des Nährmedium mit verschiedenen Konzentrationen Surfactin wurde zu den angewachsenen Zellen gegeben. Die Kulturplatte wurde weitere 2 Tage bei 37°C und in Gegenwart von 5 Vol.-% CO₂ bebrütet.
3. Die Zellen der Linien ML, Hep₂ und BHK21 waren nach 3 Tagen, die Zellen der Linien CV1 und CRFK nach 8 Tagen in den Kontrollreihen zu hohen Zelldichten herangewachsen und bildeten einen homogenen Zellrasen. Der Mediumüberstand würde abgeschlagen und zu jedem Ansatz 50 µl Kristallviolettlösung pipettiert. Die Kristallviolettlösung setzte sich zusammen aus 3,75 g Kristallviolett, 1,75 g NaCl, 161,5 ml Ethanol abs., 43,2 ml 37%igem Formaldehyd (alle Chemikalien von Merck) ad 500 ml deionisiertes und destilliertes Wasser. Die Kristallviolettlösung wurde nach 20 min intensiv aus den Kavitäten mit deionisiertem Wasser herausgewaschen und die Mikrotiterplatte an der Luft getrocknet.
4. Der verbleibende zellgebundene Farbstoff wurde durch Zugabe von 100 µl einer Lösung aus 50% Ethanol abs., 0,1% Eisessig, 49,9% deionisiertem und destilliertem Wasser je Kavitäte in Lösung gebracht und durch intensives Schütteln über 5 Minuten homogen verteilt. Die photometrische Bestimmung der Farbintensität, die in linearem Zusammenhang zur Zelldichte steht, erfolgte bei 550 nm (EAR 400 AT, SLT-Labinstruments). Es wurde die Konzentration ermittelt, die eine Reduzierung der Zellzahl um 50% bewirkte.

Es konnte für das Surfactin ein zytotoxischer Effekt von 50% bei einer Mediumkonzentration von 48 µM für die Hep₂-, von 37 µM für die BHK21-, von 45 µM für die CRFKund von 43 µM für die ML-Zellen ermittelt werden. Die CRFK- und Hep₂-Zellen zeigten bei Konzentrationen bis zu 30 µM Surfactin keine Veränderungen am Zellwachstum. Bei den ML- und BHK21-Zellen wurde bei dieser Surfactin-Konzentration eine Wachstumsverringerung um 15% beobachtet. Die in Beispiel 1 angewandte Konzentration von 40 µM Surfactin bewirkte in Langzeituntersuchungen eine Reduzierung der Zelldichte auf 15% bei den CRFK- und Hep₂-Zellen, auf 45% bei den ML-Zellen und auf 57% bei den BHK21-Zellen im Vergleich zu unbehandelten Kontrollansätzen. Keine der getesteten Kulturen tolerierte höhere Surfactin-Konzentrationen als 65 µM. Alle getesteten Zellkulturen tolerieren die Anwesenheit des Lipopeptids Surfactin über den für eine Inaktivierung von lipidumhüllten Viren notwendigen kurzen Zeitraum.

### Beispiel 10

### Biotechnologische Anwendung Inaktivierung von umhüllten Viren in Zellkulturen

Mit Hilfe der Gentechnik kann jede beliebige Zellinie potentiell wertvolle Substanzen wie Interferone, Wachstumsfaktoren, u.a. produzieren. Diese Zellkulturen können unter Umständen mit verschiedenen Virusspezies kontaminiert sein. Viruskontaminationen sollten daher schon in der Ausgangszellkultur entfernt werden. Als Beispiel wird hier die Eliminierung eines Herpesvirus aus einer Nerz-Lungenzellkultur beschrieben. Schweine-Herpesvirus Typ 1 (SHV-1) induziert einen deutlichen zytopathogenen Effekt, an dessen Ausbleiben der Inaktivierungserfolg direkt sichtbar wird.
1. Ca. 1x10⁴ frisch trypsinierte ML-Zellen (Nerz-Lungenzellen) wurden in einer Petrischale (10 cm Ø, Nunc) mit Dulbecco's modifiziertem Eagle's Medium (ICN) mit 5% (v/v) über 30 min bei 56°C inaktiviertem fötalem Kälberserum (GIBCO) passagiert. Das Zellkulturmedium wurde von den angewachsenen Zellen entfernt und diese mit ca. 100 TCID₅₀ SHV-1 infiziert. Nach einer einstündigen Inkubation wurde das Inoculum abgenommen und der Zellrasen mit 10 ml Dulbecco's modifiziertem Eagle's Medium (ICN) mit 5% (v/v) fötalem Kälberserum (GIBCO) und 50 µM Surfactin überschichtet. Das Surfactin wurde dazu in einer Konzentration von 1 mM in PBS gelöst und autoklaviert (123°C über 23 min).
2. Die ML-Zellkultur wurde 3 Tage bei 37°C und 5 Vol.-% CO₂ bebrütet. Die Zellen bedeckten 30% des Petrischalenbodens und konnten mit 0,25% Trypsin, 2 µM EDTA, abgelöst werden. Alle Zellen wurden erneut in eine Petrischale (10 cm Ø, Nunc) mit Dulbecco's modifiziertem Eagle's Medium (ICN) mit 5% (v/v) über 30 min bei 56°C inaktiviertem fötalem Kälberserum (GIBCO) und 40 µM Surfactin passagiert. Die Behandlung wurde zweimal wiederholt.
3. Nach anschließender zehnmaliger Passage ohne Surfactin wurde 1 ml des Zellkulturüberstandes auf eine frische ML-Kultur gegeben und diese nach 7 Tagen Inkubation auf einen Virus-bedingten zytopathogenen Effekt (CPE) mit dem Lichtmikroskop untersucht.

In den ML-Zellkulturen wurde kein zytopathogener Effekt als Marker der Virusvermehrung beobachtet.

### Beispiel 11

### Produktsicherheit

### Inaktivierung von SIV in einer Albuminlösung

Serum enthält eine Vielzahl von wirtschaftlich und medizinisch bedeutsamen Bestandteilen, z. B. Hormone, Immunglobuline, Gerinnungsfaktoren, Enzyme, Cholesterine, Lipoproteine, Albumine u.a.. Serum kann mit organischen Lösungsmitteln, wie Ethanol, Ether, Polyethylenglycol bei niedrigen Temperaturen und durch Präzipitation mit Salzen oder pH-Veränderungen fraktioniert und so die Bestandteile aufgereinigt werden. In Blut vorkommende Viren, wie das Retrovirus HIV, können jedoch als infektiöse Partikel in das aufgereinigte Blutprodukt gelangen. Als Beispiel für eine sich notwendigerweise anschließende Virusinaktivierung wurde Rinderserumalbumin (BSA) mit Surfactin behandelt. Zur Demonstration des Wirkungspotentiales wurde das Albumin mit einer Dosis des Affenimmundefizienzvirus (SIV) versetzt, die unter natürlichen Bedingungen nicht gefunden wird, und zeitabhängig die Anzahl der infektiösen Viruspartikel durch Aussaat auf frische Wirtszellen (Endstufentitration) bestimmt.
1. Affenimmundefizienzvirus (SIV) wurde in 50 ml PBS mit 50 mg/ml BSA und 80 µM Surfactin (gelöst in PBS in einer Konzentration von 1 mM und autoklaviert) aufgenommen. Der Anfangstiter betrug 1,6x10⁵ TCID₅₀/ml. Der pH-Wert des Inaktivierungsansatzes betrug während der Versuchsdauer durch Zugabe von 1 N HCl konstant pH 7,8 und die Temperatur 22°C.
2. Aus dem permanent gerührten Inaktivierungsansatz wurden zu den Zeitpunkten 5, 10, 15, 30, 45, 60, 90, 120 und 180 Minuten Aliquots entnommen. Von diesen Aliquots wurde aus einer 1:10 Vorverdünnung heraus eine Verdünnungsreihe zur Basis 3 erstellt. Die Verdünnungen wurden auf eine 96-Loch-Flachboden-Mikrotiterplatte (Nunc) in 8 parallele Reihen mit jeweils 100 µl einer jeden Verdünnungsstufe überführt. Die 96 Löcher der Mikrotiterplatte waren zuvor mit jeweils 100 µl einer Molt4, Klon 8-Zell-Suspension mit einer Zelldichte von 5x10⁴ Zellen/ml beschickt worden.
3. Die Platten wurden 14 Tage bei 37°C und 5 Vol.-% CO₂ bebrütet. Die Zellen in den Kontrollansätzen ohne Virusverdünnung waren zu hohen Dichten herangewachsen. Die Zellkulturen der Mikrotiterplatte wurden lichtmikroskopisch untersucht. Jede Kultur mit Anzeichen eines zytopathogenen Effektes wurde als infiziert gewertet.
4. Die Titer wurden als 50%ige infektiöse Dosis (TCID₅₀) nach der Methode von Spearman und Kärber [in: Biometrie. Grundzüge biologisch-medizinischer Statistik. (1974) Ed. L. Cavalli-Sforza. Gustav Fischer-Verlag Stuttgart S. 171-173] berechnet und auf 1 ml des Inaktivierungsansatzes bezogen.

Innerhalb von 15 Minuten reduzierte sich der Anfangstiter von 1,6x10⁵ TCID₅₀ auf eine Restinfektiösität von 1,5x10³ TCID₅₀. Nach 60 Minuten Inkubationszeit betrug der Titer an infektiösem SIV 7 TCID₅₀. Nach 120 Minuten konnten keine infektiösen Partikel mehr gefunden werden.

### Beispiel 12

### Produktsicherheit Kombinierte Anwendung von Surfactin und feuchter Hitze zur Inaktivierung von SIV in einer Albuminlösung

Blut- und biotechnologisch erzeugte Produkte werden im isolierten und gereinigten Zustand häufig einer Hitzebehandlung (Pasteurisierung) unterzogen. Diese Hitzebehandlung kann mit einem SD-Verfahren kombiniert werden. Surfactin ist aufgrund seiner Thermostabilität ebenfalls in Kombination mit einem Hitzeinaktivierungsverfahren einsetzbar. Das Beispiel beschreibt die Inaktivierung des Affenimmundefizienzvirus (SIV) in Serumalbumin zur Demonstration des synergistischen Effektes von Hitze und Surfactin bei der Virusinaktivierung.
1. Affenimmundefizienzvirus (SIV) wurde in 50 ml PBS mit 50 mg/ml BSA und 80 µM Surfactin (gelöst in PBS in einer Konzentration von 1 mM und autoklaviert) aufgenommen. Der Anfangstiter betrug 1,6x10⁵ TCID₅₀/ml. Der pH-Wert des Inaktivierungsansatzes betrug während der Versuchsdauer durch Zugabe von 1 N HCl konstant pH 7,8. Vor der Zugabe der Virussuspension wurde der Ansatz in einem Wasserbad auf 60°C vortemperiert und während des Experiments konstant gehalten.
2. Aus dem permanent gerührten Inaktivierungsansatz wurden zu den Zeitpunkten 2, 5, 10, 15, 20, 30, 45, und 60 Minuten Aliquots entnommen. Von diesen Aliquots wurde aus einer 1:10 Vorverdünnung heraus eine Verdünnungsreihe zur Basis 3 erstellt. Die Verdünnungen wurden auf eine 96-Loch-Flachboden-Mikrotiterplatte (Nunc) in 8 parallele Reihen mit jeweils 100 µl einer jeden Verdünnungsstufe überführt. Die 96 Löcher der Mikrotiterplatte waren zuvor mit jeweils 100 µl einer Molt4, Klon 8-Zell-Suspension mit einer Zelldichte von 5x10⁴ Zellen/ml beschickt worden.
3. Die Platten wurden 14 Tage bei 37°C und 5 Vol.-% CO₂ bebrütet. Die Zellen in den Kontrollansätzen ohne Virusverdünnung waren zu hohen Dichten herangewachsen. Die Zellkulturen der Mikrotiterplatte wurden lichtmikroskopisch untersucht. Jede Kultur mit Anzeichen eines zytopathogenen Effektes wurden als infiziert gewertet.
4. Die Titer wurden als 50%ige infektiöse Dosis (TCID₅₀) nach der Methode von Spearman und Kärber [in: Biometrie. Grundzüge biologisch-medizinischer Statistik. (1974) Ed. L. Cavalli-Sforza. Gustav Fischer-Verlag Stuttgart S. 171-173] berechnet und auf 1 ml des Inaktivierungsansatzes bezogen.

Innerhalb von 5 Minuten reduzierte sich der Anfangstiter von 1,6x10⁵ TCID₅₀ auf eine Restinfektiösität von 9,3x10² TCID₅₀. Nach einer 20 minütigen Inkubation konnten keine infektiösen Partikel mehr gefunden werden. Beim kombinierten Hitze/Surfactin-Verfahren wurde die Infektiösität des eingebrachten SIV mindestens um einem Faktor 10 schneller inaktiviert als durch die gleiche Surfactinkonzentration bei Raumtemperatur oder durch Hitzebehandlung ohne antivirale Zusätze.

### Beispiel 13

### Maximale Inaktivierungsraten:

Für drei Virusfamilien wurden die maximal erreichbaren Inaktivierungsraten bestimmt. Die Inaktivierungsbedingungen waren: pH 7,8; 80 µM Surfactin; in wässrigem Medium mit 5% fötalem Kälberserum; gerührt; 22°C.

| Virus | Abnahme der Infektiösität [log₁₀ TCID₅₀^{#}] | | | |
|---|---|---|---|---|
| | nach 5 min | nach 30 min | nach 60 min | nach 120 min |
| Herpesviren | 2,30 | 4,90 | 5,40 | - |
| Retroviren | 1,30 | 2,90 | 4,40 | - |
| Rhabdoviren | 1,00 | 3,60 | 4,50 | 5,40 |

| | | | | |
|---|---|---|---|---|
| # 50% Gewebekultur infizierende Dosis | | | | |

## Patentansprüche

1. Verfahren zur Inaktivierung von lipidumhüllten Viren in biologischen oder biotechnologischen Produkten oder in Zellkulturen,
**dadurch gekennzeichnet, daß**
den Produkten oder Zellkulturen als Inaktivierungsagens ein zyklisches Lipopeptid, dessen Salze oder Ester oder Gemische davon zugesetzt und die Inaktivierung bei Raumtemperatur innerhalb von 30 Minuten bis maximal 2 Stunden durchgeführt wird, wobei
a) bei der Inaktivierung von Viren in den Produkten das Agens den Produkten in einer Konzentration von 1-100µM zugesetzt wird oder
b) bei der Inaktivierung von Viren in den Zellkulturen dem serumfreien Kulturmedium das Agens in einer Konzentration von 1-65µm oder dem serumhaltigen Kulturmedium in einer Konzentration von 10-100µM zugesetzt wird.

2. Verfahren nach Anspruch 1,
**dadurch gekennzeichnet,**
**daß** die Virusinaktivierung in biologischen oder biotechnologischen Produkten bei höheren Temperaturen als Raumtemperatur, vorzugsweise 30 - 60°C, innerhalb von 5 - 30 min durchgeführt wird.

3. Verfahren nach Anspruch 1 oder 2,
**dadurch gekennzeichnet,**
**daß** als zyklische Lipopeptide natürlich vorkommende, chemisch synthetisierte, gentechnisch hergestellte oder gentechnisch modifizierte Lipopeptide eingesetzt werden.

4. Verfahren nach einem der Ansprüche 1-3,
**dadurch gekennzeichnet,**
**daß** als Lipopeptide Lipoheptapeptide der allgemeinen Formel I 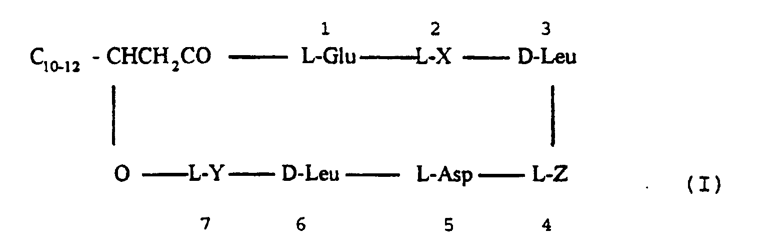 deren Salze oder Ester oder deren Gemische eingesetzt werden, wobei in Formel I X und Y unabhängig voneinander für die Aminosäuren Leu, Ile oder Val und Z für die Aminosäuren Val oder Ala steht und C₁₀₋₁₂ eine lineare oder verzweigte, gesättigte Alkylkette bedeutet.

5. Verfahren nach Anspruch 4,
**dadurch gekennzeichnet,**
**daß** Verbindungen der allgemeinen Formel I mit C₁₁-Alkyl oder C₁₂-Alkyl eingesetzt werden.

6. Verfahren nach Anspruch 4 oder 5,
**dadurch gekennzeichnet,**
**daß** die Ester der Verbindungen der allgemeinen Formel I eingesetz werden, vorzugsweise die Monoester.

7. Verfahren nach einem der Ansprüche 4 bis 6,
**dadurch gekennzeichnet,**
**daß** Verbindungen der allgemeinen Formel I mit X und Y in der Bedeutung von Leu und Z in der Bedeutung von Val eingesetzt werden.

8. Verfahren nach einem der Ansprüche 4 bis 6,
**dadurch gekennzeichnet,**
**daß** Verbindungen der allgemeinen Formel I mit X in der Bedeutung von Ile oder Val eingesetzt werden.

9. Verfahren nach einem der Ansprüche 1 bis 8,
**dadurch gekennzeichnet,**
**daß** lipidumhüllte humane und animale Viren inaktiviert werden.

10. Verfahren nach einem der Ansprüche 1 bis 8,
**dadurch gekennzeichnet,**
**daß** Herpes-Viren, vorzugsweise HSV-1, HSV-2, BHV-1, SHV-1, Imundefizienz-Viren, vorzugsweise HIV-1, HIV-2, SIV_{agm}, das vesikuläre Stomatitis-Virus (VSV) und das Semliki-Forest-Virus (SFV) inaktiviert werden.

11. Lipoheptapeptide der allgemeinen Formel I 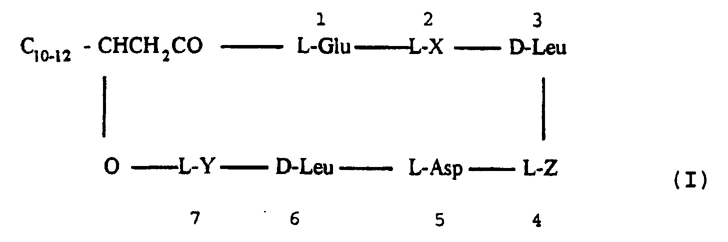 sowie deren Salze und Ester, wobei in Formel I X und Y unabhängig voneinander Val oder Ile bedeuten und Z Val ist.

12. Verwendung der Lipopeptide gemäß Ansruch 11 zur Inaktivierung von lipidumhüllten Viren in biologischen oder biotechnologischen Produkten oder in Zellkulturen.

## Claims

1. A method of inactivating lipid-enveloped viruses in biological or biotechnological products or in cell cultures, **characterized in that** the products or cell cultures are added with a cyclic lipopeptide, salts or esters thereof, or mixtures of same as inactivating agent, and inactivation is performed at room temperature within from 30 minutes up to 2 hours at maximum, wherein
a) the agent is added to said products at a concentration of 1-100 µM when inactivating viruses in the products;
b) the agent is added to the serum-free culture medium at a concentration of 1-65 µM, or to the serum-containing culture medium at a concentration of 10-100 µM, when inactivating viruses in cell cultures.

2. The method of claim 1, **characterized in that** the virus inactivation in biological or biotechnological products is performed at temperatures higher than room temperature, preferably 30-60°C, within a period of 5-30 min.

3. The method according to claim 1 or 2, **characterized in that** naturally occurring, chemically synthesized lipopeptides, as well as those produced or modified by genetic engineering are used as cyclic lipopeptides.

4. The method according to any of claims 1-3, **characterized in that** lipoheptapeptides of general formula I, 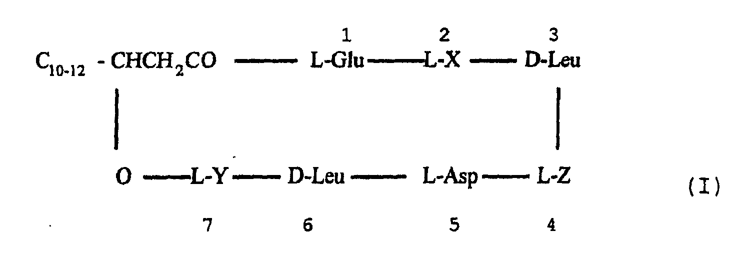 the salts, esters or mixtures thereof are used as lipopeptides, in which formula I X and Y independently represent the amino acids Leu, Ile or Val, Z represents the amino acids Val or Ala, and C₁₀₋₁₂ represents a linear or branched, saturated alkyl chain.

5. The method according to claim 4, **characterized in that** compounds of general formula I with C₁₁ alkyl or C₁₂ alkyl are used.

6. The method according to claim 4 or 5, **characterized in that** esters of the compounds of general formula I, preferably monoesters are employed.

7. The method according to any of claims 4-6, **characterized in that** compounds of general formula I are used, wherein X and Y represent Leu, and Z represents Val.

8. The method according to any of claims 4-6, **characterized in that** compounds of general formula I are used, wherein X represents Ile or Val.

9. The method according to any of claims 1-8, **characterized in that** lipid-enveloped human and animal viruses are inactivated.

10. The method according to any of claims 1-8, **characterized in that** herpes viruses, preferably HSV-1, HSV-2, BHV-1, SHV-1, immunodeficiency viruses, preferably HIV-1, HIV-2, SIV_{agm}, the vesicular stomatitis virus (VSV), and the Semliki-Forest virus (SFV) are inactivated.

11. Lipoheptapeptides of general formula I 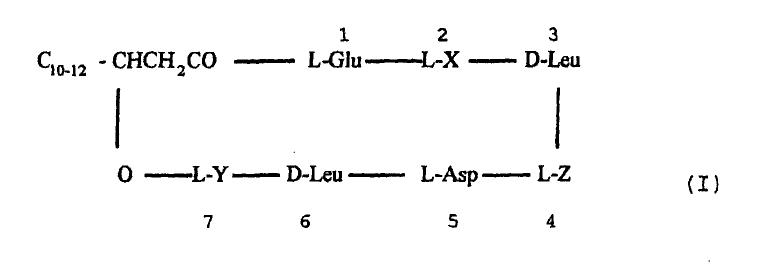 and the salts and esters thereof, in which formula I X and Y independently represent Val or Ile, and Z represents Val.

12. Use of the lipopeptides according to claim 11 for inactivating lipid-enveloped viruses in biological or biotechnological products, or in cell cultures.

## Revendications

1. Procédé pour l'inactivation de virus lipogainés dans des produits biologiques ou biotechnologiques ou dans des cultures cellulaires,
**caractérisé en ce qu'**
est ajouté comme agent d'inactivation aux produits ou aux cultures cellulaires un lipopeptide cyclique, ses sels, esters ou des mélanges de celui-ci, et que l'inactivation est effectuée à température ambiante dans une période comprise entre 30 minutes et 2 heures maximum,
a) l'agent étant ajouté aux produits dans une concentration de 1 à 100 µM pour l'inactivation de virus dans les produits, ou bien,
b) pour l'inactivation de virus dans les cultures cellulaires, l'agent étant ajouté dans une concentration de 1 à 65 µM à un milieu de culture exempt de sérum, ou dans une concentration de 10 à 100 µM à un milieu de culture contenant du sérum.

2. Procédé selon la revendication 1,
**caractérisé en ce que**
l'inactivation de virus à températures élevées dans des produits biologiques ou biotechnologiques est effectuée aux températures supérieures à la température ambiante, préférentiellement de 30 à 60°C, dans une période comprise entre 5 et 30 minutes.

3. Procédé selon revendication 1 ou 2,
**caractérisé en ce que**
sont pris comme lipopeptides cycliques des lipopeptides obtenus naturellement, par synthèse chimique, produits par génie génétique ou modifiés en recourant au génie génétique.

4. Procédé selon l'une des revendications 1 à 3,
**caractérisé en ce que**
sont pris comme lipopeptides des lipoheptapeptides de la formule générale 1, 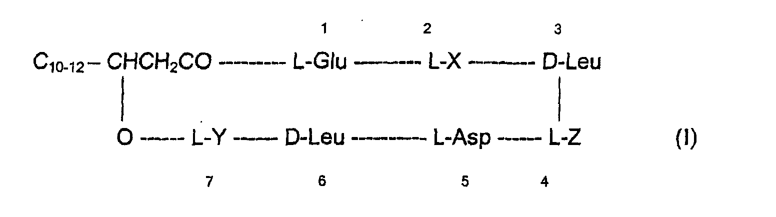 leurs sels, esters ou leurs mélanges, X et Y représentant dans la formule 1, indépendamment l'un de l'autre, les acides aminés Leu, lie ou Val, Z représentant les acides aminés Val ou Ala, et C₁₀₋₁₂ se référant à une chaîne alkyle linéaire ou ramifiée, saturée.

5. Procédé selon la revendication 5,
**caractérisé en ce qu'**
il est recouru à des composés de la formule générale 1 avec de l'alkyle C₁₁ ou de l'alkyle C₁₂.

6. Procédé selon revendication 4 ou 5,
**caractérisé en ce qu'**
il est recouru aux esters des composés de la formule générale 1, préférentiellement les monoesters.

7. Procédé selon l'une des revendications 4 à 6,
**caractérisé en ce qu'**
il est recouru à des composés de la formule générale I avec X et Y représentant Leu et Z représentant Val.

8. Procédé selon l'une des revendications 4 à 6,
**caractérisé en ce qu'**
il est recouru à des composés de la formule générale I avec X représentant Ile ou Val.

9. Procédé selon l'une des revendications 1 à 8,
**caractérisé en ce que**
des virus humains et animaux lipogainés sont inactivés.

10. Procédé selon l'une des revendications 1 à 8,
**caractérisé en ce que**
des virus herpétiques, préférentiellement HSV-1, HSV-2, BHV-1, SHV-1, des virus immunodéficitaires, préférentiellement HIV-1, HIV-2, SIV_{agm}, le virus de la stomatite vésiculaire (VSV) et le virus de la fièvre virale de Semliki (SFV) sont inactivés.

11. Lipoheptapeptides de la formule générale 1, 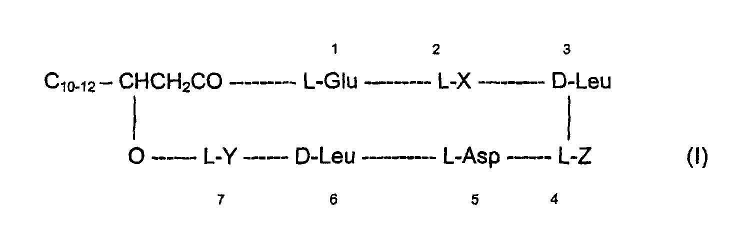 ainsi que leurs sels et esters, X et Y représentant dans la formule 1, indépendamment l'un de l'autre, Val ou Ile, et Z représentant Val.

12. Recours à des lipopeptides selon revendication 11 pour l'inactivation de virus lipogainés dans des produits biologiques ou biotechnologiques ou dans des cultures cellulaires.
